(19) Europäisches Patentamt
European Patent Office
Office européen des brevets

(11) EP 2 636 307 B1

(12) EUROPEAN PATENT SPECIFICATION

(45) Date of publication and mention
of the grant of the patent:
**07.01.2015 Bulletin 2015/02**

(51) Int Cl.:
*A01N 37/02* (2006.01)      *A23K 1/16* (2006.01)
*A23K 1/18* (2006.01)      *A61K 31/20* (2006.01)
*C11C 1/04* (2006.01)      *A01P 1/00* (2006.01)

(21) Application number: **12001554.0**

(22) Date of filing: **07.03.2012**

(54) **The method for production of antimicrobial compostion containing free fatty acids**

Verfahren zur Herstellung von antimikrobieller Zusammensetzung enthaltend freie Fettsäuren

Procédé de production de composition antimicrobienne contenant d'acides gras libres

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**11.09.2013 Bulletin 2013/37**

(73) Proprietor: **Cargill, Incorporated**
**Wayzata, MN 55391 (US)**

(72) Inventors:
• **Lobee, Henricus, Wilhelmus, Jozef**
**5305 CD Zuilichem (NL)**
• **Kruidenberg, Marcus, Bernardus**
**3233 SL Oostvoorne (NL)**
• **Hollander, Frank**
**4051 BL Ochten (NL)**

(74) Representative: **Dottridge, Cass A.C. et al**
**Cargill Europe bvba**
**Bedrijvenlaan 9**
**2800 Mechelen (BE)**

(56) References cited:
**US-A- 4 223 040          US-A- 5 142 071**
**US-A1- 2012 041 065**

• **Anonymous: "Exporter of distilled coconut fatty acid", ECPlaza , 4 October 2008 (2008-10-04), XP002681815, Retrieved from the Internet: URL: http://www.ecplaza.net/trade-leads-sel ler/ exporter-of-distilled-coconut-fatty--5 352705.html [retrieved on 2012-08-13]**

• **Anonymous: "Edenor HK 8-18", Oleochemicals , 21 May 2007 (2007-05-21), pages 1-2, XP055035357, Retrieved from the Internet: URL: http://cognis.plansbiz.net/imgs/upload s/1file__ 08201003728.pdf [retrieved on 2012-08-13]**

• **Gregorio C Gervajio: "Fatty Acids and Derivatives from Coconut" In: "Bailey's Industrial Oil and Fat Products", 15 July 2005 (2005-07-15), John Wiley & Sons, Inc., XP055035497, pages 1-55, * pages 8-13 ***

• **WIM DE GREYT: "Developments in Edible Oil Refining for the Production of High Quality Food Oils", 101ST AOCS ANNUAL MEETING, 19 May 2010 (2010-05-19), XP055035474, Phoenix, Arizona**

• **'Specification of "Palomys" hydrolyzed vegetable oil', [Online] 02 October 2010, page W1A, XP055112753 Retrieved from the Internet: <URL:http://www.elancocentral.com/Palomys.p df> [retrieved on 2014-04-08]**

• **'Lauric acid min. 98% - Food-grade flavor ingredient', [Online] XP055112945 Retrieved from the Internet: <URL:http: //www.sigmaaldrich.com/catalog/pr oduct/ aldrich/w261408?lang=en&region=US> [retrieved on 2014-04-08]**

• **'Lauric acid min. 98% - Food-grade flavor ingredient', [Online] 08 April 2014, pages 1 - 2, XP055112945 Retrieved from the Internet: <URL: http://www.sigmaaldrich.com/catalog/pr oduct/ aldrich/w261408?lang=en&region=US> [retrieved on 2014-04-08]**

## Description

### TECHNICAL FIELD

The present invention relates to a method of producing antimicrobial compositions, in particular compositions which can be used to prevent the growth of microbial agents in animals

### BACKGROUND OF THE INVENTION

Medium-chain fatty acids (MCFAs), i.e. fatty acids with a carbon chain length from 6-12 carbon atoms, are considered to be a unique category of fat substances. Unlike long-chain fatty acids, MCFAs can be absorbed directly into the bloodstream without re-esterification or inclusion in chylomicrons. As such, MCFAs can be transported rapidly to organs requiring energy. What's more, MCFAs are preferentially oxidized in the mitochondria, making them an excellent source of fast energy.

Certain MCFAs have also been found to have a beneficial antimicrobial effect. This is considered a key attribute in the field of animal husbandry where controlling levels of microorganisms in the animals' digestive tract is a priority. Animals may be subject to exposure to bacteria, yeast and fungi through the rearing environment and feed products. These microorganisms can cause significant disruption to the animals' digestive system and an imbalance in the microbial ecosystem of their gastrointestinal tract. This can result in less efficient digestion and nutrient absorption which, in turn, will affect growth rates. It could also lead, in some cases, to disease and, potentially, to the loss of the animal. In any event, it is clear that being able to control microbial populations has a significant effect on profitability. This used to be achieved through the application of low-dose antibiotics. However, the addition of such growth promoters to feed products was banned, in the EU, in 2006. Interest in natural alternatives - such as MCFAs - has therefore increased.

Lauric oils, such as coconut oil and palm kernel oil, are known to be rich in MCFAs. Unfortunately, triglycerides containing these fatty acids do not have the observed antimicrobial activity themselves. The MCFAs have to be separated from their glycerol backbone and used in their free fatty acid (FFA) form. This is achieved, in the industry, by splitting crude lauric oils and then distilling the oil to obtain a FFA fraction and a glycerine fraction.

There are at least four known methods of fat splitting: the Twitchell process (although somewhat archaic now), the batch autoclave process, the continuous countercurrent process, and the enzymatic process (using lipase enzymes). The fatty acids produced by these processes are then purified and separated into fractions by distillation and fractionation.

Because of the extreme sensitivity of fatty acids to heat, oxidation and corrosion effects, distillation must be performed under highly controlled conditions - i.e. under high vacuum, at lower temperatures and with the shortest possible residence time. Nonetheless, fatty acid distillates tend to develop a strong taste and smell (caused by secondary oxidation products such as ketones and aldehydes). They can also have a strong degree of coloration due to the presence of pigments such as carotenoids. This process may also result in concentrations of contaminants (such as dioxins and poly aromatic hydrocarbons (PAHs)) which are very difficult to remove.

Nonetheless, these distillates have been considered generally acceptable for animal feed. There is, however, increasing concern over contaminant levels. What's more, their strong odor and taste has always made them unsuitable for use in certain animal feeds: aquatic feed (e.g. for fish and shrimp), feed for young animals (e.g. veal calves and piglets), and domestic animal food all require the use of less strong-tasting and -smelling oils.

"Exporter distilled coconut fatty acid, URL:http://www.ecplaza.net/trade-leads-seller/exporter-of-distilled-coconut-fatty--5352705 describes a distilled coconut fatty acid with Lovibond cell red of max.

Oelochemicals: "Edenor HK 8-18", URL:http://cognis.plansbiz.net/imgs/uploads/&file_08201003728.pdf describes cocnut fatty acid with a Lovibond 0.2 red.

US 2012/0041065 relates to lauric acid distillate for animal feed.

US 5,142,071 describes the selective esterification of long chain fatty acid monoglycerides with medium chain fatty acids.

Gregorio C. Gervajio in Bailey's Industrial Oil and Fat products, Sixth edition - Fatty Acids and Derivatives from Coconut Oil describes the manufacture and uses of coconut fatty acids.

Wim De Greyt, in developments in Edible Oil Refining for the Production of High Quality Food Ils, 101st AOCS Annual Meeting 2010, describes the edible oil refining process.

US 4,223,040 describes lauric acid for the prevention and treatment of mycobacterial diseases. There is therefore a clear need in the art for a method of producing an antimicrobial composition with better or less taste, smell and color and with a reduced contaminant content. The present invention addresses this need.

### STATEMENTS OF THE INVENTION

According to a first aspect of the present invention, there is provided a method of producing an antimicrobial composition, the method comprising the step of splitting a refined lauric oil to obtain a composition comprising 90% or more, by weight, free fatty acids, characterized in that the refined oil is an oil that has been brought into contact with activated carbon.

## DESCRIPTION OF INVENTION

The present invention provides a method of producing antimicrobial compositions. The term "antimicrobial" as used herein refers to substances that are capable of killing or inhibiting the growth of microorganisms such as bacteria and fungi (including yeasts). In particular, it may refer to substances that are capable of killing or inhibiting the growth of fungi such as Aspergillus, Candida, Cephalosporum, Fusarium, and Penicillium; yeasts such as Saccharomyces; Gram-negative bacteria such as Escherichia coli, Salmonella, and Shigella; and Gram-positive bacteria such as Listeria.

The process of the present invention includes the step of splitting a refined lauric oil to obtain a composition comprising 90% or more, by weight, free fatty acids, characterized in that the refined oil is an oil that has been brought into contact with activated carbon. Lauric oils will generally be understood to be oils rich in MCFAs, i.e. in C6-C12 fatty acids. Examples of such oils include coconut oil, palm kernel oil, babassu oil, cohune oil, tacum oil and cuphea oil. For the purposes of the present invention, the lauric oil will preferably be selected from coconut oil and palm kernel oil, or mixtures thereof.

Crude lauric oils - i.e. as extracted from their original source - will typically not be suitable for use in the present process due to the presence of high levels of contaminants - such as phosphatides, soaps and pigments - which may cause an undesirable colour, odor or taste. As such they will first be refined.
Refining typically consists of three major steps: degumming, bleaching and deodorization, all of which will be well known processes to a person skilled in the art. Thus, the method of the present invention will preferably use lauric oils which have been subjected to at least degumming, bleaching and deodorization. Other refining steps may also be used. The refined lauric oils used in the present method will have been treated (or brought into contact) with activated carbon.

Activated carbon treatment may be performed with powdered, granular, extruded or bead activated carbon - or with any other known form of activated carbon. Preferably, it will be performed in a column with granular activated carbon. It may be performed separately from the other refining steps. Alternatively, it may be performed simultaneously with the bleaching step (e.g. by combining the activated carbon and a bleaching earth together in a single column). The oil will preferably be brought into contact with the activated carbon at a temperature between 50-120°C, preferably between 50-90°C. For example, the oil may be brought into contact with the activated carbon at a temperature of about 60°C. The activated carbon may be used, for instance, in an amount of 10g per MT of oil to 50 kg per MT of oil. According to one embodiment, it will be used in an amount of 1-2 kg per MT of oil. Other parameters and variations thereof will be apparent to a person skilled in the art.

According to the present method, a refined lauric oil, brought into contact with activated carbon, is split to obtain a composition comprising 90% or more, by weight, free fatty acids. Splitting, as described above, is a process for separating fatty acids from their glycerol backbone. A number of splitting techniques are known in the art and do not all need to be described in great detail here. For instance, the splitting technique used in the method of the present invention may consist of chemical hydrolysis and/or enzymatic splitting. By way of illustration only, chemical hydrolysis may be performed in a batch process (e.g. in a batch autoclave process) or in continuous process (also known as the Colgate-Emery process). Enzymatic splitting will preferably be performed with lipase enzymes such as those from Candida Rugosa, Aspergillus niger or Rhizopus arrhizus.

Preferably, splitting will be achieved through chemical hydrolysis under high pressure and at high temperatures. The obtained compositions will have a FFA content of at least 90% by weight and will have an appropriate profile for use as natural antimicrobial agents in the manufacture of animal feeds.

FFA content is measured using the standard method AOCS Ca 5a-40 (97). Preferably, the composition will comprise 95% or more, more preferably 97% or more, more preferably 99% or more FFA by weight. The FFAs will have a high MCFA content. Preferably, they will comprise more than 50% MCFAs. More preferably, they will comprise 60% or more, 70% or more, or 80% or more MCFAs.

The term "MCFA" as used herein will be understood, in the context of the present invention, as referring to fatty acids with a carbon chain length from 6-12 carbon atoms (i.e. caproic acid (C6), caprylic acid (C8), capric acid (C10) and lauric acid (C12)), as well as to salts, derivatives, emulsions and mixtures thereof. A composition may comprise, by weight:

- 0-5%, preferably 0-1 %, C6 fatty acids;
- 1-15% , preferably 2-10%, C8 fatty acids;
- 1-15%, preferably 1-8%, C10 fatty acids; and
- 35-70%, preferably 40-55%, C12 fatty acids.

The term "refined antimicrobial composition" refers to the fact that the compositions will have less color, a better taste and/or smell, and/or fewer contaminants (that is to say a lower concentration of contaminants) than similar antimicrobial compositions obtained by distillation. In particular, the compositions will have:

- a Lovibond red colour of 5 or less; and/or
- an acceptable taste and smell as determined by Method A; and/or
- a total PAH level of 20 TEQ B[a]P or less; and/or
- a total dioxin content no greater than 1.5 TEQ.

Ideally, they will have a Lovibond red colour of 5 or less; and an acceptable taste and smell as determined by Method A; and a total PAH level of 20 TEQ B[a]P or less; and a total dioxin content no greater than 1.5 TEQ.

Lovibond red colour is measured on the Lovibond colour scale in accordance with AOCS method Cc13e Red color is graded on a scale of 0.1 to 20. Preferably, oils obtained according to the method of the present invention will have a Lovibond Red colour of 5 or less, more preferably of 2 or less, more preferably of 1 or less, for example of 0.5 or less. Ideally, the oils will be white (when in their solid state).

They will also preferably be devoid of any burnt taste or smell (which is often associated with e.g. coconut oil due to the processes used to dry the source materials). Taste and odor are assessed according to Method A: AOCS Method Cg 2-83. According to this method, oils are given a score from 1 to 10, wherein 1 indicates an oil with a very strong taste and/or odor, and 10 indicates a tasteless, odorless oil. Compositions will be considered as having an acceptable taste and odor if, when measured according to Method A, they have a score of 7 or more. Preferably, the compositions will be characterized by a score of 8 or more.

Advantageously, the compositions will also have very low levels of contaminants. In particular, they will preferably have reduced levels of polycyclic aromatic hydrocarbons (PAHs) and of dioxins and dioxin-like compounds (DLCs) compared to corresponding oils obtained by distillation and fractionation. For ease of reference, dioxins and DLCs will commonly be referred to herein simply as dioxins.

As toxic compounds all have a different toxic effects, toxicity is typically measured in terms of toxicity equivalence (TEQ). TEQ is calculated as the product of the concentration of an individual toxic compound in a sample (in $\mu$g/kg) and its corresponding toxicity equivalency factor (TEF), i.e. as follows:

$$\boxed{\text{TEQ of compound A = concentration of A x TEF of A}}$$

The TEF of a compound is expressed as a number, between 0 and 1, which represents the compound's relative toxicity compared to that of the most toxic known compound in that category of compounds. Thus, for instance, the TEFs for PAHs are calculated relative to the toxicity of benzo[a]pyrene - and the PAH content of a composition will therefore be expressed in terms of TEQ B[a]P. The TEFs of dioxins are calculated relative to the toxicity of 2,3,7,8-tetrachlorodibenzodioxin (TCDD) - with the dioxin content of a composition conventionally being expressed simply in terms of "TEQ".

The toxicity of a composition - i.e. its total toxicity equivalence - will depend, to a certain extent, on the number of PAHs or dioxins being measured. Regulatory guidelines and food and feed law in different countries will often dictate the number of compounds that must be taken into account when assessing a product's toxicity. For instance, the composition will preferably have a total PAH content of no more than 20 TEQ B[a]P, wherein this total TEQ value corresponds to the sum of TEQs for four specific PAHs, namely benzo[a]pyrene, benzo[a]anthracene, benzo[b]fluoranthene and chrysene (this is referred to as the 4-PAH method - PAH content can also be measured based on 11 or 12 PAHs, i.e. using the 11-PAH or 12-PAH methods). In particular, when the antimicrobial composition is produced from coconut oil, the total PAH content may be up to 20 TEQ B[a]P. If it is produced from palm kernel oil, it will preferably be no higher than 10 TEQ B[a]P. Preferably, the composition will have a total PAH content of no more than 10, more preferably no more than 4, more preferably no more than 1 TEQ B[a]P. In addition, the composition will preferably have a B[a]P content of no more than 2, more preferably no more than 1 TEQ.

Its total dioxin content will preferably be no more than 1.5, more preferably no more than 1 TEQ - corresponding to the sum of TEQs for polychlorinated biphenyls (PCB), polychlorinated dibenzo-p-dioxins (PCDD) and polychlorinated dibenzofurans (PCDF). Furthermore, the composition will preferably have a PCB level no higher than 0.75, more preferably no higher than 0.5 TEQ, and a PCDD+PCDF level preferably no higher than 0.75, more preferably no higher than 0.5 TEQ.

The compositions are suitable for use as antimicrobial agents for animal feed.

The term "animal feed" as used herein, includes all solid or semi-solid feeds as well as liquid feeds and premixes. The animal feed will be admixed with the composition mentioned before to form an animal feed which, when administered, will provide an effective amount of the composition to the animal.

**EP 2 636 307 B1**

Feeds (or feed compositions) are comprising the above antimicrobial compositions in an amount of 0.05 to 10% by weight, preferably in an amount of 0.2 to 5% by weight, even more preferably in an amount of 0.3 to 2% by weight, for example in an amount of 0.5 to 1 % by weight.

In addition to the antimicrobial composition, the animal feeds may further comprise one or more other active ingredients. These may include any material which can be added to the feed to enhance the animal's health, performance, and/or well-being. Examples of such ingredients are referred to in "2006 Feed Additive Compendium" and "Handbook of Feed Additives 2006".

Feed compositions may be used, for instance, for equine animals (such as horses), ovine animals (such as lamb and sheep) and bovine animals (such as cattle), but will be particularly suitable for calves (e.g. veal calves), porcine animals (such as pigs and piglets), rabbits, poultry (such as chickens, turkeys, ducks, pheasant and quail), domestic animals (such as cats and dogs), and aquatic animals (such as fish and shrimp).

The antimicrobial compositions, and animal feeds comprising them, may be used to enhance feed efficiency and/or enhance growth and/or reduce mortality in animals.

Feed efficiency is a term generally known in the art and refers to a ratio of weight of food ingested/weight gain of an animal. Enhancement of feed efficiency is an overall decrease of said ratio.

Growth and enhancing growth are terms generally known in the art and refer to increases in either, or both, weight and size (e.g., height, width, diameter, circumference, etc.).

Growth can refer to an increase in the mass (e.g., weight or size) of the entire animal or of a particular tissue (e.g., muscle tissue in general or a specific muscle). Alternatively, growth can indicate a relative increase in the mass of one tissue in relation to another, in particular, an increase in muscle tissue relative to other tissues (e.g., adipose tissue).

Reducing mortality refers to increasing the survivability or decreasing the death rate in animals after birth or hatch.

Effective amount refers to the amounts of administration of the antimicrobial composition, to provide enhanced growth, enhanced feed efficiency, and/or reduced mortality. Further, such amount and rates should result in no or few adverse events in the treated animal. As those familiar with the art will understand, the amounts and rates will vary depending upon a number of factors. These factors include, for example, the type of animal being treated, its weight and general physical condition, and the dosing regimen. Ranges for the rate of administration of the antimicrobial composition are from about 1 to about 3000, desirably 10 to 1000, and more desirably from about 10 to about 500 mg/kg of weight of the animal. These amounts are to be administered normally every day for at least 7 days, at least 2 weeks, at least 30 days, over 60 days, over 100 days, or for all or a substantial portion of the life of the animal.

**Claims**

1. A method of producing an antimicrobial composition, the method comprising the step of splitting a refined lauric oil to obtain a composition comprising 90% or more, by weight, free fatty acids, **characterized in that** the refined oil is an oil that has been brought into contact with activated carbon.

2. A method according to claim 1, **characterized in that** the refined oil is an oil that has been degummed, bleached and deodorized.

3. A method according to any one of the preceding claims, **characterized in that** the splitting step is selected from chemical hydrolysis and enzymatic splitting.

4. A method according to any one of the preceding claims, **characterized in that** the lauric oil is selected from coconut oil, palm kernel oil and mixtures thereof.

**Patentansprüche**

1. Verfahren zum Herstellen einer antimikrobiellen Zusammensetzung, wobei das Verfahren den Schritt des Spaltens eines raffinierten Laurinöls umfasst, um eine Zusammensetzung zu erhalten, die 90 Gew.-% oder mehr freie Fettsäuren umfasst, **dadurch gekennzeichnet, dass** das raffinierte Öl ein Öl ist, dass mit Aktivkohle in Kontakt gebracht wurde.

2. Verfahren nach Anspruch 1, **dadurch gekennzeichnet, dass** das raffinierte Öl ein Öl ist, dass entschleimt, gebleicht und desodoriert wurde.

**3.** Verfahren nach einem der vorherigen Ansprüche, **dadurch gekennzeichnet, dass** der Spaltschritt aus chemischer Hydrolyse und enzymatischer Spaltung ausgewählt ist.

**4.** Verfahren nach einem der vorherigen Ansprüche, **dadurch gekennzeichnet, dass** das Laurinöl aus Kokosnussöl, Palmkernöl und Mischungen davon ausgewählt ist.

**Revendications**

**1.** Procédé de production d'une composition antimicrobienne, le procédé comprenant l'étape de clivage d'une huile laurique raffinée pour obtenir une composition comprenant 90 % ou plus en poids d'acides gras libres, **caractérisé en ce que** l'huile raffinée est une huile qui a été mise en contact avec du charbon activé.

**2.** Procédé selon la revendication 1, **caractérisé en ce que** l'huile raffinée est une huile qui a été dégommée, blanchie et désodorisée.

**3.** Procédé selon l'une quelconque des revendications précédentes, **caractérisé en ce que** l'étape de clivage est choisie entre l'hydrolyse chimique et le clivage enzymatique.

**4.** Procédé selon l'une quelconque des revendications précédentes, **caractérisé en ce que** l'huile laurique est choisie entre l'huile de noix de coco, l'huile de palmiste et leurs mélanges.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120041065 A
- US 5142071 A

- US 4223040 A

**Non-patent literature cited in the description**

- *Feed Additive Compendium,* 2006

- Handbook of Feed Additives. 2006